# EUROPÄISCHE PATENTANMELDUNG

(11) **EP 1 726 305 A1**
(43) Veröffentlichungstag der Anmeldung: **29.11.2006**
(21) Anmeldenummer: 05011361.2
(22) Anmeldetag: 25.05.2005
(51) Int. Cl.: A61K 31/4439, A61P 1/04, A61K 33/30, C07D 401/12

(54) **Zinksalz des Omeprazols und dessen Enantiomere**

(71) Anmelder: Ratiopharm GmbH, 89079 Ulm (DE)
(72) Erfinder: Stahl, Heinrich.Dr., D-79104 Freiburg (DE)
(74) Vertreter: Best, Michael

(57) **Zusammenfassung**

Die Erfindung betrifft Zinksalz des Omeprazols. Die neuen Verbindungen haben eine sehr hohe Schüttdichte, eine sehr hohe Stampfdichte und eine gegenüber insbesondere dem bekannten Magnesiumsalz verringerte Löslichkeit. Sie eignen sich besonders gut für die Herstellung von Retardformulierungen.

## Beschreibung

Die Erfindung betrifft neue Salze des Arzneistoffs Omeprazol und dessen Enantiomere, insbesondere ein Salz des (-)-Enantiomers des Omeprazols. Bei den neuen Salzen handelt es sich um Zinksalze.

Zink ist ein allgegenwärtiges Element, das für die normale enzymatische Funktion im Metabolismus essentiell ist. Zinkchlorid wird beispielsweise als Arzneimittel mit adstringierenden Eigenschaften verwendet. Zink wird ebenfalls als Stabilisator in Insulinpräparaten verwendet. Zink wird auch in höheren Dosen als nicht toxisch angesehen.

Omeprazol ist ein seit langem bekannter Wirkstoff mit einer Hemmwirkung gegen die Magensäuresekretion und eignet sich daher als Therapeutikum insbesondere zur Behandlung von Magengeschwüren. Omeprazol ist temperaturempfindlich, lichtempfindlich und besonders empfindlich gegenüber Feuchtigkeit und sauren pH-Werten. Das (-)-Enantiomer des Omeprazols ist beispielsweise aus der DE-A 40 35 455 bekannt, wird auch als Esomeprazol bezeichnet und zeigt prinzipiell die gleichen Probleme.

Alkalische Salze des Omeprazols sind z.B. in der EP-A 124 495 offenbart. Die Salze zeigen zwar eine erhöhte Lagerstabilität gegenüber der Neutralform des Omeprazols; Fertigarzneimittel mit Omeprazol oder einem Omeprazolsalz zeigen dennoch häufig nicht die gewünschte Stabilität. Zur Stabilisierung des Omeprazols in Fertigarzneimitteln wurden eine Reihe von Vorschlägen gemacht, beispielsweise in dem EP 247 983, das vorschlägt, Omeprazol mit einer alkalischen Verbindung zu formulieren oder ein alkalisches Salz des Omeprazols zu verwenden, das Arzneimittel mit einer enterischen Beschichtung zu versehen und den alkalisch reagierenden Kern von der notwendigerweise sauer reagierenden enterischen Beschichtung durch eine im wesentlichen neutrale Zwischenschicht zu trennen.

Eine Reihe von Druckschriften schlägt darüber hinaus vor, dem Omeprazol zur Stabilisierung in einem Fertigarzneimittel in einem Wirkstoffkern ein stabilisierend wirkendes Zusatzmittel beizufügen.

Ähnliche Stabilitätsprobleme zeigen auch andere Benzimidazolderivate, und daher sind verschiedenste Gemische aus Benzimidazolderivaten mit anderen Verbindungen im Stand der Technik bekannt. Die EP-A 1 018 340 offenbart z.B. stabile Einschlußverbindungen von Benzimidazolderivaten mit Aminosäuresalzen und Cyclodextrin. Das Benzimidazolderivat kann auch als Aminosäuresalz, insbesondere als L-Argininsalz, vorliegen, allerdings werden in der EP-A 1 018 340 ausschließlich saure Salze des Omeprazols, also Salze des Omeprazolkations, mit einem Aminosäureanion offenbart. Entsprechend offenbart die WO 00/012064 die Stabilisierung von Omeprazol mit Arginin oder Lysin in der anionischen Form, die als pH-Stabilisatoren dienen. Die WO 99/56756 lehrt, Omeprazol gemeinsam mit Fluoridionen zur Behandlung von Helicobacter pylori zu verabreichen, wobei in einer Ausführungsform die Fluoridionen als Zinkfluorid zur Verfügung gestellt werden. Die WO 99/53918 offenbart, daß ein Benzimidazolderivat oder ein Alkalisalz davon durch eine Reihe von Zusatzstoffen, darunter auch beispielsweise Arginin und Aspartat, stabilisiert wird. Die WO 98/40069 beschreibt die Stabilisierung eines Cyclodextrin-Omeprazol-Einschlußkomplexes mit einer Aminosäure. Mittel gegen Magengeschwüre mit einem Benzimidazol und einer Aminosäure können auch der JP 05194225 entnommen werden. Die EP-A 444 625 nennt Omeprazolformulierungen für die rektale Anwendung, die mit einer basischen Aminosäure als Stabilisator formuliert werden.

Die WO 94/27988 offenbart, daß bestimmte Salze des Esomeprazols, nämlich das Natriumsalz, das Magnesiumsalz, das Lithiumsalz, das Kaliumsalz, das Calciumsalz und Ammoniumsalze verbesserte pharmakokinetische und metabolische Eigenschaften haben mit einem verbesserten therapeutischen Profil wie einem geringeren Grad an interindividueller Variation.

In WO 03/074514 werden zusätzlich noch die entsprechenden Monoalkylammoniumsalze mit ähnlichen Eigenschaften offenbart.

WO 04/037253 und WO2004/020436 offenbaren amorphe Formen der im vorhergehenden Abschnitt genannten Salze des Esomeprazols.

Weitere polymorphe Formen des Esomeprazol-Magnesiumsalzes werden in WO 04/046134 und WO2004/089935 offenbart.

Derzeit wird in Form von Tabletten vor allem das Magnesiumsalz des (-)-Enantiomers des Omeprazols unter der Bezeichnung NEXIUM vermarktet. Das Magnesiumsalz des Esomeprazols weist in wäßrigen Lösungsmitteln, insbesondere Wasser oder Darmsaft, eine Löslichkeit auf, die eine relativ schnelle Freisetzung des Esomeprazols nach der Verabreichung ermöglicht. Für die Entwicklung von Retardarzneimitteln ist die schnelle Freisetzung des Magnesiumsalzes des Omeprazols bzw. des Esomeprazols jedoch nicht vorteilhaft.

Das Magnesiumsalz des Esomeprazols bzw. des Omeprazols weist ebenso bei der Verarbeitung zu Tabletten eine Reihe von Nachteilen auf. So hat es eine geringe Schüttdichte (Bulk-Density) und eine geringe Stampfdichte (Tap-Density) d.h. relativ schlechte Fließeigenschaften. Bei der Verarbeitung neigt es zur elektrostatischen Aufladung und haftet daher an Behälterwänden. Das Magnesiumsalz des Esomeprazols bzw. des Omeprazols hat relativ schlechte Gleit- bzw. Schmiereigenschaften und als Trihydrat einen hohen Kristallwassergehalt, was wegen der Wasserempfindlichkeit des Omeprazols inhärent ungünstig ist. Unter den Bedingungen des obersten Dünndarms wird das Magnesiumsalz des Esomeprazols relativ schnell aufgelöst und unterliegt während der Passage des obersten Dünndarms wegen des sauren bis neutralen pH-Wertes einer erheblichen Zersetzung.

Es besteht damit ein Bedürfnis nach neuen Formen des Omeprazols und insbesondere des Esomeprazols, die die vorstehenden Probleme nicht zeigen, die insbesondere durch ihre höhere Schütt- und Stampfdichte und geringere elektrostatische Aufladbarkeit leichter verarbeitbar sind als das Magnesiumsalz. Die neuen Formen des Omeprazols bzw. des Esomeprazols sollten einen geringen Wassergehalt aufweisen und eine bessere Stabilität insbesondere im schwach sauren Bereich des obersten Dünndarms aufweisen.

Darüber hinaus sollten die neuen Formen des Omeprazols bzw. des Esomeprazols eine Freisetzungscharakteristik zeigen, die sie besonders geeignet für die Verwendung in Retardarzneimitteln macht.

Diese Aufgaben werden durch den Gegenstand der Patentansprüche gelöst.

Die Erfindung betrifft insbesondere die Verbindungen Ia, Ib and Ic:
Verbindung Ia: Zinksalz des racemischen Omeprazols
Verbindung Ib: Zinksalz des (-)-Enantiomers von Omeprazol
Verbindung Ic: Zinksalz des (+)-Enantiomers von Omeprazol

Ein weiterer Aspekt der vorliegenden Erfindung ist ein Verfahren zur Herstellung der Zinksalze von Omeprazol und der Enantiomere des Omeprazols. Ein solches Verfahren kann prinzipiell auf dem Fachmann bekannten Verfahren basieren, wie es in den Beispielen gezeigt ist.

Schließlich betrifft die Erfindung auch Retardarzneimittel mit den neuen Salzen.

Erfindungsgemäß wurde überraschend gefunden, daß das Zinksalz des Omeprazols, insbesondere des (-)-Enantiomers des Omeprazols nicht nur eine gegenüber dem Magnesiumsalz stark verringerte Löslichkeit aufweist, sondern darüber hinaus auch überraschenderweise eine erheblich höhere Schüttdichte und Stampfdichte zeigt. Das Zinksalz des Omeprazols bzw. seiner Enantiomere und insbesondere des Esomeprazols hat eine bessere Fließfähigkeit als das Magnesiumsalz. Bei der Tablettierung haben die Zinksalze gegenüber den Magnesiumsalzen verbesserte Gleit- und Schmiereigenschaften und zeigen kaum elektrostatische Aufladung und damit auch kaum die Tendenz an Behälterwänden zu haften. Insgesamt ergibt sich damit gegenüber den Magnesiumsalzen insbesondere bei der Tablettierung eine überraschend bessere Verarbeitbarkeit.

Das erfindungsgemäße Zinksalz des (-)-Enantiomers des Omeprazols hat eine sehr hohe Schüttdichte von bevorzugt mehr als 350 g/l, stärker bevorzugt mehr als 400 g/l. Auch die Stampfdichte des erfindungsgemäßen Zinksalzes des (-)-Enantiomers des Omeprazols ist erheblich höher als die Stampfdichte des Magnesiumsalzes, bevorzugt 300 g/l oder mehr, stärker bevorzugt 400 g/l oder mehr, am stärksten bevorzugt 450 g/l oder mehr. Besonders bevorzugt sind die in den Beispielen angegebenen Werte für die Schüttdichte und die Stampfdichte.

Sofern nichts anderes angegeben ist, erfolgt die Bestimmung der Schüttdichte und Stampfdichte nach Ph.Eur. 5.0 General Notices 2.9.15.

Insbesondere wurde zur Bestimmung der Schüttdichte jeweils etwa 250 ml Substanz, möglichst erschütterungsfrei in einen 250 ml Meßzylinder des Schütt-Stampf-Volumeters (Erweka SVM 22, Ser. Nr. 108.480.446) gefüllt und die Masse bestimmt (Schüttgewicht). Anschließend wurde der Meßzylinder 1250 Stampfvorgängen ausgesetzt, und das Stampfvolumen wurde abgelesen. Die Werte für die Schütt- bzw. Stampfdichte erhält man durch Division der Einwaage durch die jeweiligen Volumenwerte. Die Bestimmung wurde dreifach durchgeführt und aus den Einzelergebnissen jeweils der Mittelwert gebildet.
Die Löslichkeit wurde bei Zimmertemperatur (25°C) bei verschiedenen pH-Werten untersucht, indem festes Material in den jeweiligen Medien durch Ultraschall und Schütteln soweit als möglich gelöst wurde. Die Proben wurden filtriert und über HPLC analysiert und mittels UV-Detektion vermessen.

Während das Magnesiumsalz als Trihydrat vorliegt und daher per se einen erheblichen Wassergehalt von etwa 6,23% aufweist, ist der Wassergehalt des Zinksalzes des (-)-Enantiomers des Omeprazols ausgesprochen gering und liegt bei nur etwa 0,8%, was ebenfalls einen überraschenden Vorteil des Zinksalzes des (-)-Enantiomers des Omeprazols ausmacht. Diese Messungen erfolgten mittels Karl-Fischer-Titration gemäß Ph.Eur. 2.5.32 mit dem Gerät Aqua 40.00 (Fa. ECH Halle). Das Gerät war ausgestattet mit einem Ausheizofen für Feststoffproben. Die Ausheiztemperatur für die untersuchte substanz betrug 130 °C: Als KF-Reagenz wurde eingesetzt Hydranal (Coulomat AG, Riedel-de Haen. Als Standard wurde verwendet Natriumtartrat-dihydrat (Apura, Merck).

Das Zinksalz des Omeprazols bzw. seiner Enantiomere und insbesondere des Esomeprazols kann auf an sich bekannte Art und Weise hergestellt werden. Hierzu wird beispielsweise eine starke Base, insbesondere eine Alkalibase, wie Natriumhydroxid, Kaliumhydroxid oder Lithiumhydroxid, bevorzugt Kaliumhydroxid, in Wasser gelöst und bevorzugt von Sauerstoff und Kohlendioxid befreit. Anschließend wird das Omeprazol bzw. das Enantiomer zugesetzt. Ein lösliches Zinksalz wie Zinkchlorid oder bevorzugt Zinkacetat wird zugegeben. Das Zinksalz fällt aus und kann abfiltriert und auf übliche Art und Weise aufgereinigt werden.

Überraschenderweise ist das Zinksalz des Omeprazols bzw. eines Enantiomeren davon und insbesondere des Esomeprazols auch besonders zur Herstellung von Retardformulierungen geeignet, da es aus Arzneimitteln nur sehr langsam aber im wesentlichen kontinuierlich freigesetzt wird. Die vorliegende Erfindung betrifft daher auch Retardformulierungen, die das Zinksalz des Omeprazols oder eines Enantiomeren davon, insbesondere das Zinksalz des Esomeprazols enthalten. Retardformulierungen werden auch als Arzneiformen mit protrahierter Wirkung bezeichnet und sind beispielsweise in Hunnius, Pharmazeutisches Wörterbuch, 8te Auflage 1998 definiert und auf diese Literaturstelle wird insoweit Bezug genommen. Die Herstellung von Retardformulierungen ist im Stand der Technik beschrieben und gehört zum allgemeinen Fachwissen eines Fachmanns.

Ein weiterer Vorteil der vorliegenden Erfindung besteht auch darin, daß anders als bei dem Magnesiumsalz des Omeprazols bzw. des Esomeprazols bei der Verabreichung von Arzneimitteln mit dem Zinksalz des Omeprazols bzw. eines Enantiomeren davon, insbesondere mit dem Zinksalz des Esomeprazols, nur ein geringer Anteil des Wirkstoffs in den obersten Dünndarmbereich freigesetzt wird, in dem häufig noch ein saurer pH-Wert herrscht, der den säurelabilen Wirkstoff zersetzen kann, bevor er resorbiert wird. Die Freisetzung des Wirkstoffs erfolgt vielmehr langsam und kontinuierlich, so daß insgesamt weniger Wirkstoff im Körper zersetzt wird, bevor er resorbiert wird.

Die folgenden Beispiele erläutern die Erfindung.

### Beispiel 1

### Herstellung des Zinksalzes des (-)-Enantiomers des Omeprazols

5,6 g Kaliumhydroxid wurden in 190 ml Kohlendioxid-freies Wasser gelöst. Die Lösung wurde mit Argon gespült. 35 g des (-)-Enantiomers des Omeprazols (Esomeprazol, (-)-5-Methoxy-2-[[(4-methoxy-3,5-dimethyl-2-pyridinyl)methyl]sulfinyl]-1H-benzimidazol) wurde portionsweise zu dem Gemisch gegeben und aufgelöst. Man erhielt eine braune Lösung. Über einen Zeitraum von etwa 30 Minuten wurden 13,2 g Zinkacetat zugegeben. Es ergab sich eine cremefarbene, dicke Suspension. Die Suspension wurde über Nacht bei Raumtemperatur unter einer Argonatmosphäre gerührt, wobei sich ein rot-braunes Gemisch bildete.

Der Feststoff wurde über eine Nutsche abfiltriert und in der Nutsche mit insgesamt 300 ml Wasser suspendiert. Das Wasser wurde abgesaugt, und das Produkt wurde unter vermindertem Druck 3 bis 4 Stunden bei 40°C getrocknet. Man erhielt 42,8 g der Titelverbindung als rosa-braunen Feststoff.

Zur weiteren Aufreinigung wurde das so erhaltene Produkt bei 45°C und 250 ml Ethylacetat gelöst. Die Lösung wurde filtriert, und das Filtrat wurde auf eine Masse von 160 g eingedampft. Anschließend wurden 500 ml Methyl-tert.-butylether zugegeben. Der erhaltene Feststoff wurde abfiltriert und mit 50 ml Methyl-tert.-butylether, der 5% Ethylacetat enthielt (MtBe 5% EtOAc), gewaschen. Der nasse Feststoff wurde in 200 ml MtBe 5% EtOAc suspensiert, abfiltriert und mit 100 ml MtBe 5% EtOAc und 350 ml Methyl-tert.-butylether gewaschen. Das Produkt wurde unter vermindertem Druck 3 bis 4 Stunden bei 40°C getrocknet. Man erhielt 34,5 g (90,6%) der Titelverbindung als cremefarbenen Feststoff.

Der Feststoff wurde in 500 ml Tetrahydrofuran/Methylenchlorid gelöst. Die Lösung wurde mit 5 g Aktivkohle geklärt, es wurde filtriert, und das Filtrat wurde unter vermindertem Druck auf 150 g eingedampft. 500 ml Methyl-tert.-butylether wurden zugegeben. Das ausgefallene Produkt wurde abfiltriert und unter vermindertem Druck bei 40°C getrocknet. Man erhielt 30,5 g (80,6%) eines ockerfarbenen Feststoffs. Der Zinkgehalt wurde über Atomabsorptionsspektroskopie auf 8,8% bestimmt. Bis 230°C wurde kein Schmelzpunkt beobachtet. Die optische Reinheit wurde zu 99,64% e.e. bestimmt (chirale Chromatographie, Säule: Chiral AGP 100 x 4, Chromtech, Eluent: 15 mM Phosphatpuffer pH = 7,0 mit 10% Acetonitril (v/v)).

### Beispiel 2

### Untersuchung der Dichten

Auf folgende Art und Weise wurde die Schüttdichte und die Stampfdichte des nach Beispiel 1 hergestellten Zinksalzes des (-)-Enantiomers des Omeprazols bestimmt und mit den auf entsprechende Art und Weise bestimmten Werten des Magnesiumsalzes des (-)-Enantiomers des Omeprazols verglichen. Das Magnesiumsalz des (-)-Enantiomers des Omeprazols wurde entsprechend der Offenbarung der WO 94/27988 hergestellt.

Zur Bestimmung der Schüttdichte wurden jeweils 250 ml Substanz möglichst erschütterungsfrei in einen 250 ml Meßzylinder des Schütt-Stampf-Volumeters (Erweka SVM 22, Ser. Nr. 108.480.446) gefüllt und die Masse bestimmt (Schüttgewicht). Anschließend wurde der Meßzylinder 1250 Stampfvorgängen ausgesetzt, und das Stampfvolumen wurde abgelesen. Die Werte für die Schütt- bzw. Stampfdichte erhält man durch Division der Einwaage durch die jeweiligen Volumenwerte. Die Bestimmung wurde dreifach durchgeführt und aus den Einzelergebnissen jeweils der Mittelwert gebildet.

**Tabelle 1**

| Verbindung | Schüttdichte (g/l) | Stampfdichte (g/l) |
|---|---|---|
| Magnesiumsalz des (-)-Enantiomers des Omeprazols | 199,00 | 242,68 |
| Zinksalz des (-)-Enantiomers des Omeprazols | 415,62 | 481,59 |

Das Zinksalz des (-)-Enantiomers des Omeprazols zeigt damit bessere Fließeigenschaften als das Magnesiumsalz des (-)-Enantiomers des Omeprazols.

### Beispiel 3

### Vergleich der Löslichkeiten

Die Löslichkeit des Zinksalzes des (-)-Enantiomers des Omeprazols und des Magnesiumsalzes des (-)-Enantiomers des Omeprazols wurde wie folgt bestimmt. In den unten aufgeführten Medien wurden gesättigte Lösungen (n=1) hergestellt, die nach 30 Minuten Ultraschallbehandlung noch einen deutlichen Bodensatz zeigten. Der Überstand der gesättigten Lösungen wurde über einen Membranfilter (0,45µm, Millex HV 25 mm, Millipore) filtriert und die klare Lösung mittels einer HPLC-Methode auf den Gehalt des gelösten Salzes untersucht. (Reversed Phase Chromatographie, Gradientenmodus, Säule: Luna C18(2), 150 x 3 mm, Phenomenex, Eluent 1: 10mM Tris-(hydroxymethyl)-aminomethan-Puffer pH 9.0, Eluent 2 : Acetonitril).

Das Zinksalz des (-)-Enantiomers des Omeprazols wurde gemäß Beispiel 1 vorstehend hergestellt, das Magnesiumsalz des (-)-Enantiomers des Omeprazols gemäß der Offenbarung der WO 94/27988.

Die gemessenen Löslichkeiten können folgender Tabelle entnommen werden.

**Tabelle 2:**

| Verbindung | Puffer | Löslichkeit [mg/ml] |
|---|---|---|
| Magnesiumsalz des (-)-Enantiomers des Omeprazols | dem. Wasser | 0,93 |
| | USP-Puffer pH 6,0 | 0,78 |
| | USP-Puffer pH 6,8 | 0,9 |
| | USP-Puffer pH 7,4 | 0,89 |
| Zinksalz des (-)-Enantiomers des Omeprazols | dem. Wasser | 0,02 |
| | USP-Puffer pH 6,0 | 0,07 |
| | USP-Puffer pH 6,8 | 0,07 |
| | USP-Puffer pH 7,4 | 0,04 |

Aufgrund der geringen Löslichkeit eignet sich das Zinksalz des Omeprazols insbesondere auch für die Herstellung von Retardformulierungen.

### Beispiel 4

### Tablettierversuche

Auf bekannte Art und Weise wurden das Zinksalz des (-)-Enantiomers des Omeprazols und das Magnesiumsalz des (-)-Enantiomers des Omeprazols zu Mikrotabletten mit einem Durchmesser von 2 mm gepreßt. Die Zusammensetzung kann folgender Tabelle entnommen werden.

**Tabelle 3:**

| Inhaltsstoff | Menge (g) | Menge (%) |
|---|---|---|
| Salz des (-)-Enantiomers des Omeprazols | 10,00 | 24,88 |
| Mikrocelac 100 | 25,80 | 64,18 |
| Kollidon CL | 2,00 | 4,98 |
| Polyglykol 8000 P | 2,00 | 4,98 |
| Pruv (Natriumstearylfumarat) | 0,40 | 1,00 |
| Gesamt | 40,2 | 100 |

Das Zinksalz hatte bei der Verarbeitung wesentlich bessere Fließeigenschaften als das Magnesiumsalz. Anders als beim Magnesiumsalz des (-)-Enantiomers des Omeprazols traten keine Probleme mit einer elektrostatischen Aufladung auf, während das Magnesiumsalz des (-)-Enantiomers des Omeprazols eine sehr starke elektrostatische Aufladung zeigte. Das Zinksalz zeigte daher kaum eine Neigung, an den Behälterwänden anzuhaften. Die Schmiereigenschaften des Zinksalzes des (-)-Enantiomers des Omeprazols waren besser als die Schmiereigenschaften des Magnesiumsalzes des Omeprazols.

Das Verhältnis zwischen Preßkraft und Tablettenhärte war bei dem Zinksalz des (-)-Enantiomers des Omeprazols zufriedenstellend. Sowohl die Tabletten mit dem Magnesiumsalz als auch die Tabletten mit dem Zinksalz zerfielen unmittelbar in weniger als 10 Sekunden.

In der folgenden Tabelle wird die Freisetzung des Zinksalzes des (-)-Enantiomers des Omeprazols mit der Freisetzung des Magnesiumsalzes des (-)-Enantiomers des Omeprazols aus der in dem Beispiel hergestellten Mikrotablette verglichen. (US Pharm. XXII, S. 1578 - 1579, USP Test 711). Die Mikrotabletten wurden vor dem Auftrag einer enterischen Beschichtung vermessen.

| Zeit (min) | Freisetzung Wirkstoff (%) | |
|---|---|---|
| | Mikrotablette Magnesiumsalz | Mikrotablette Zinksalz |
| 10 | 96 | 19 |
| 20 | 98 | 32 |
| 40 | 97 | 48 |
| 60 | 97 | 58 |

## Patentansprüche

1. Zinksalz des Omeprazols oder eines Enantiomeren des Omeprazols.

2. Zinksalz nach Anspruch 1, nämlich das Zinksalz des Esomeprazols.

3. Zinksalz nach Anspruch 1 oder 2, **dadurch gekennzeichnet, daß** die Schüttdichte 350 g/l oder mehr beträgt.

4. Arzneimittel umfassend das Zinksalz des Omeprazols oder eines Enantiomeren des Omeprazols nach einem der Ansprüche 1 bis 3 und gegebenenfalls ein oder mehrere pharmazeutisch verträgliche Träger und/oder Hilfsstoffe.

5. Arzneimittel nach Anspruch 4, **dadurch gekennzeichnet, daß** es sich um ein Retardarzneimittel handelt.

6. Verwendung eines Zinksalzes nach einem der Ansprüche 1 bis 3 zur Herstellung eines Arzneimittels zur Vorbeugung und/oder Behandlung von Erkrankungen des Magen-Darmtraktes.

7. Verwendung nach Anspruch 6, wobei das Arzneimittel zur Vorbeugung und/oder Behandlung einer Erkrankung ausgewählt aus Magengeschwüren, Übersäuerung des Magens, peptisches Ulcus gastritis, gastroösophagialer Reflux (GER), Refluxösophagitis, symptomatischer gastroösophagialer Reflux, erosive Ösophagitis, Dyspepsie vom nicht-Ulcer-Typ, zur Behandlung von Helicobacter pylori, Unterdrückungen von Blutungen im oberen Verdauungskanal, die durch ein peptisches Ulcer hervorgerufen werden, akutem Streßulcer, unter Stress hervorgerufenem Ulcer nach einer Operation zur Beseitigung von Helicobacter pylori ist.
